(19) **Europäisches Patentamt**
**European Patent Office**
**Office européen des brevets**

(11) **EP 1 544 620 B1**

(12) **EUROPEAN PATENT SPECIFICATION**

(45) Date of publication and mention
of the grant of the patent:
**31.03.2010 Bulletin 2010/13**

(51) Int Cl.:
***G01N 33/68*** [(2006.01)]

(21) Application number: **04029190.8**

(22) Date of filing: **09.12.2004**

(54) **Quantitation by serial combinatorial dilution**

Quantifizierung durch kombinatorische Verdünnung

Quantification par dilution combinatoire en série

(84) Designated Contracting States:
**AT BE BG CH CY CZ DE DK EE ES FI FR GB GR HU IE IS IT LI LT LU MC NL PL PT RO SE SI SK TR**
Designated Extension States:
**LV**

(30) Priority: **18.12.2003 EP 03104775**

(43) Date of publication of application:
**22.06.2005 Bulletin 2005/25**

(73) Proprietor: **F. Hoffmann-La Roche AG**
**4070 Basel (CH)**

(72) Inventors:
• **Berndt, Peter**
**4056 Basel (CH)**

• **Evers, Stefan**
**79379 Muellheim (DE)**
• **Langen, Hanno**
**79585 Steinen (DE)**

(56) References cited:
**WO-A-95/19359**

• **WEIJLAND A ET AL: "THE PURIFICATION AND CHARACTERIZATION OF THE CATALYTIC DOMAIN OF SRC EXPRESSED IN SCHIZOSACCHAROMYCES POMBE COMPARISON OF UNPHOSPHORYLATED AND TYROSINE PHOSPHORYLATED SPECIES" EUROPEAN JOURNAL OF BIOCHEMISTRY, BERLIN, DE, vol. 240, no. 3, 1996, pages 756-764, XP001121077 ISSN: 0014-2956**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

The present invention relates to a method for the quantification of a biomolecule in a complex mixture of biomolecules comprising fractionation of the complex mixture into fractions with subsequent serial combinatorial dilution of the fractions and detection of the biomolecules in each original fraction and each diluted fraction by a method with a defined sensitivity threshold and identification capabilities.

A current method for detection of a biomolecule (for example a protein) is the two dimensional gel electrophoresis with subsequent volumetric analysis of the stained gel. However, it is difficult to determine the quantity of the analyzed biomolecule, especially if its quantities in different samples shall be compared. To account for the inter-sample variation in biomolecule concentrations the gels have to be processed in parallel and a gel-to-gel-matching has to be done.

There is a need of a simple method for quantification of biomolecules in a complex mixture of biomolecules.

The present invention provides a method for the quantification of a biomolecule in a complex mixture of biomolecules comprising

    a. providing at least two fractions derived from the fractionation of the complex mixture of biomolecules comprising each at least one distinct biomolecule component,

    b. subjecting the fractions to a serial combinatorial dilution step, until the concentrations of the biomolecule fall below the sensitivity threshold of the subsequent detection and identification method

    c. detecting and identifying the biomolecule in each original fraction and each diluted fraction by a method providing a sensitivity threshold and identity information, to determine the presence or absence of each biomolecule in each of the original and diluted fractions, and

    d. quantifying the biomolecule in the complex mixture of biomolecules by summarizing the number of identifications of the biomolecule in each fraction on each dilution level in consideration of the respective dilution factor.

For the purpose of normalization the sum of d) may be divided by the total number of identifications of all biomolecules in all fractions on all dilution levels (dilution levels of original fractions and diluted fractions).

The method of the present invention for the quantification of a biomolecule provides a relative quantification of one or more biomolecules in a complex mixture of biomolecules from one source compared to the respective biomolecules in a complex mixtures from other sources.

The complex mixture of biomolecules may be derived from any source comprising biological sources comprising cells, cell culture supernatants, biological fluids such as serum, plasma, urine, bronchial lavage fluid, sputum, biopsies like cerebrospinal fluid. The complex mixture of biomolecules comprises at least two different biomolecules. The biomolecule in the present invention may be any biomolecule comprising polynucleotides, polypeptides, carbohydrates, lipids, glycoproteins, lipoproteins or other modified forms or metabolites thereof. The detection and identification method can be restricted to a single type of biomolecules, or can detect and analyze several classes of biomolecules at one time.

The fractionation method used in the method of the present invention should efficiently separate the complex mixture of biomolecules into distinct fractions. Preferably, the complex mixture of biomolecules is fractionated into distinct fractions with each different biomolecule only being present in not more than n minus one fractions wherein n is the total number of fractions and n is equal or higher than two. Preferably, the different biomolecules are present in two different fractions, more preferably in one fraction. The fractionation method which may be used in the method of the present invention may be selected from any method suitable for separation of a complex mixture of the targeted type of biomolecules. The fractionation method which may be used in the method of the present invention may be selected from the group comprising fractionation based on adsorption, gravity or sedimentation velocity, electrophoretic fractionation or combinations of these methodologies. For example, in the case of proteins as the target molecule the group includes but is not limited to chromatographic fractionation, ultracentrifugation, protein precipitation, affinity purification, or immunoprecipitation. In the case of peptides (for example obtained from proteolytic digests) as the target molecule the group includes but is not limited to high pressure liquid chromatography (HPLC).

The fractions are then subjected to a serial combinatorial dilution. The serial combinatorial dilution requires at least two fractions to start with. Preferably, the complex mixture of biomolecules is fractionated in as many fractions as necessary to allow a detection and identification of a sufficient number of different targeted biomolecules in the subsequent detection step. Preferably, the number of original fractions is not a prime number, more preferably the number of original fractions is even, and preferably, each initial fraction comprises at least one distinct component.

The number of the fractions to start with the serial combinatorial dilution is dependent on the complexity of the mixture of biomolecules, on the concentration of the individual biomolecules in the complex mixture of biomolecules, the efficiency of the separation methodology, and on the detection and identification method used after the fractionation and the serial combinatorial dilution.

For the serial combinatorial dilution at least two different fractions containing at least one different biomolecule are combined. Preferably, two fractions are combined. This will change the concentration of a biomolecule in the pooled fraction according to the quotient of the dot product of the concentration of the biomolecule in each fraction with the volume of each fraction by the total volume of all combined fractions. In general, this will result in a smaller concentration of any biomolecular component in the diluted fraction compared with the maximum concentration of the respective biomolecule in the original fractions. In the following dilution steps, the concentrations of the individual proteins decrease till they fall below the sensitivity threshold of the subsequent detection and identification method.

The number of dilution steps depends on the starting concentration of the biomolecules in the original fractions, the number of original fractions after fractionation and the detection limit of the detection and identification method.

The method of the present invention further comprises a detection and identification method. The detection method has to feature a defined sensitivity threshold and to provide identity information about the detected biomolecule. Thereby, the presence or absence of a specific biomolecule can be determined in an original fraction or a diluted fraction. The detection and identification method of the present invention may rely on the chemical composition, structure, or sequence of the biomolecule and the physico-chemical or enzymatic properties resulting therefrom. These include hybridization with a specific probe, reaction with a specific antibody or lectin, enzymatic or chemical reaction with a specific molecular probe, isoelectric point, molecular weight, molecular masses of fragments resulting from enzymatic digestion of the biomolecule, NMR spectrum or combinations thereof. For the example of protein quantitation, the detection and identification method of the present invention may be selected from the group comprising combinations of one- or two-dimensional gel electrophoresis with mess spectrometry, immunoassays (e.g. western blot), gas chromatography combined with mess spectrometry (GS/MS) or electrophoresis with specifically labeled molecular entities, e.g. fluorescent, chemical (e.g. biotin), or radioactive tags.

To derive the quantitation of a biomolecule the identifications or the specific fingerprints (peptide mass fingerprints) of the fractions of each dilution step are calculated whereby the respective dilution factor for each dilution step is considered. The resulting number of identifications of the biomolecule is summarized for all dilution levels. For the purpose of normalization this sum may be divided by the total number of identifications of all biomolecules in all fractions (original fractions and diluted fractions).

$$\text{Relative Quantity (q)} = \frac{\sum (d_i \times N_i)}{N_{total}}$$

wherein $N_i$ is the number N of identifications of an individual biomolecule at dilution level i, $d_i$ the dilution factor d of the respective dilution level i and $N_{total}$ the total number N of identifications of all biomolecules in all fractions on all dilution levels.

This method is independent of the properties of the various biomolecules. Polynucleotides may be processed as well as polypeptides or carbohydrates. A further advantage of this method is that it combines quantification with the identification of a biomolecule in a simple manner without the need for additional efforts targeted at biomolecule quantitation. Moreover, if the quantity of a biomolecule derived of one source shall be compared with the one of another source the mixtures of biomolecules may processed separately of each other.

Having now generally described this invention, the same will become better understood by reference to the specific examples, which are included herein for purpose of illustration only and are not intended to be limiting unless otherwise specified, in connection with the following figures.

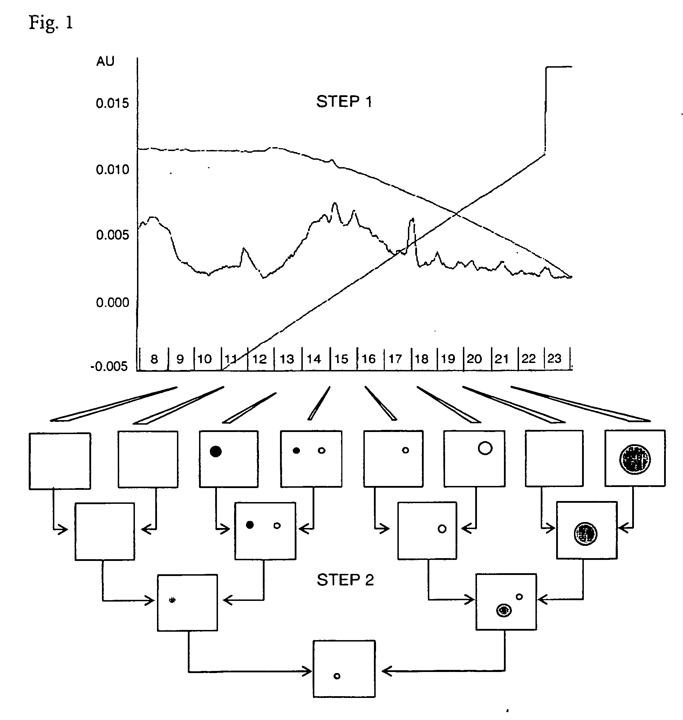

Figure 1 illustrates the method of the present invention: In a first step the complex mixture is fractionated into different fractions. These fractions are then subjected to a serial combinatorial dilution. In a second step a biomolecule is detected by for example two dimensional gel electrophoresis on the sample pools with subsequent mass spectrometric identification.(AU: Absorption Unit; 8 to 23: Fractions)

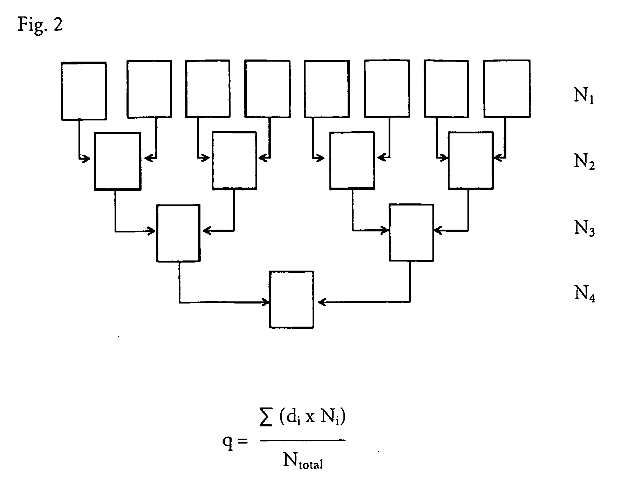

Figure 2 shows the calculation the relative quantity of a biomolecule. q: relative quantity of a biomolecule; $N_i$: the number N of identifications of an individual biomolecule on dilution level i; $d_i$: the dilution factor d of the respective dilution level i; $N_{total}$: the total number N of identifications of all biomolecules in all fractions on all dilution levels. (Scheme: N1: undiluted, N2: 2-fold dilution, N3: 4-fold dilution, N4: 8-fold dilution)

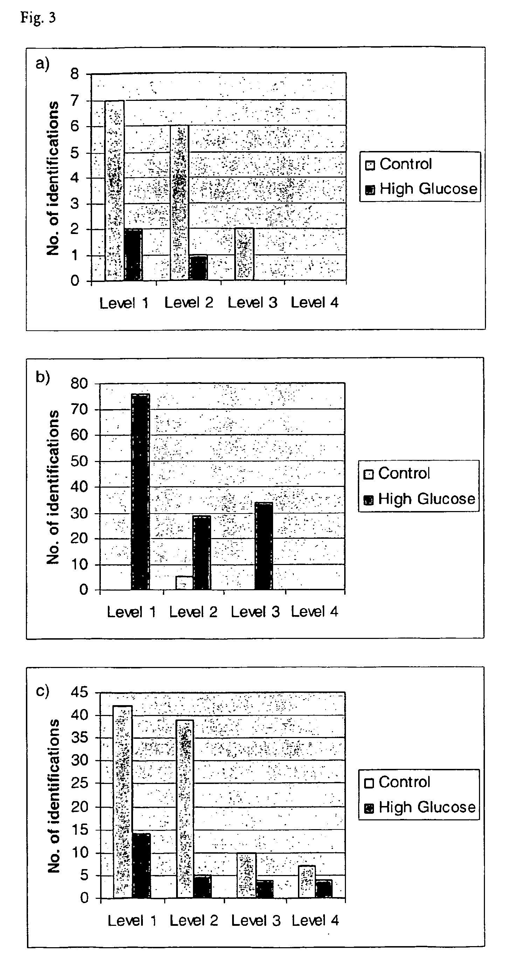

Figure 3 shows the number of identifications for the proteins glycogen phosphorylase (a), vimentin (b)and the heat shock protein 105 (c) in two dimensional electrophoresis gels from level 1 (no dilution), level 2 (2-fold dilution), level 3 (4-fold dilution), and level 4 (8-fold dilution). The values were added up from experiments carried out in triplicate. (Control: 5mM Glucose; high Glucose: 10mM)

The document in European Journal Biochemistry 1996, 240, Pages 756-764 describes serial combination to pool active fractions and not for quantitation purposes.

Examples

Commercially available reagents referred to in the examples were used according to manufacturer's instructions unless otherwise indicated.

Example 1:

*Cell Culture*

INS-1 cells (Asfari, Janjic et al. 1992) were cultured in RPMI 1640 medium (Invitrogen) supplemented with 10% FCS (Invitrogen, heat inactivated) 10 mM Hepes solution(Invitrogen), 1 mM Na pyruvate (Sigma) ; 50 $\mu$M beta-mercaptoethanol (Sigma), 1% Penicillin / Streptomycin solution (SIGMA), and low (5 mM) or high (10 mM) concentrations of glucose (SIGMA). Cells were generally cultivated at low glucose concentrations. For preparative culture, the cells were split and then incubated in low-glucose medium until cells were confluent. The medium was then changed to either low-glucose or high-glucose medium and incubations were continued for four days. For harvesting, cells were first washed once with Hanks Balanced Salt Solutions (HBSS, Invitrogen) and then covered with Trypsin/EDTA solution for 1 - 2 min until cells become rounded and detach from the bottle surface. The Trypsin/EDTA solution was discarded and the cells were suspended in Trypsin Inhibitor Solution (SIGMA), transferred to centrifuge tubes and centrifuged at 1200 x g for 5 min. After this, the cells were washed three times in HBSS, again using the same centrifuge parameters. The supernatant was aspired and discarded and the pellet was stored frozen at -80°C until used for the preparation of cytosol.

*Preparation of Cytosol*

All solutions were cooled to 4°C (except for HBSS) and all steps were carried out in a cooled environment (ice bath). Ca $10^8$ cells were resuspended in cell homogenization medium (CHM; 150 mM MgCl2, 10 mM KCl, 10 mM Tris, 0.25 M glucose, 1 mM EDTA, pH 7.4) and left on ice for 2 min. The cell suspension was transferred to a Potter-Elvehjem homogenization vessel. The cold pestle of a Potter-Elvehjem homogenizer was attached to an overhead high-torque electric motor and the cells were homogenized using 10 strokes at 1000 rpm. The efficiency of the homogenization (> 90% of broken cells) was confirmed by phase-contrast microscopy. Cell debris and nuclei were removed by centrifuging for 5 min at 1000 x g. The mitochondria were separated by centrifugation at 5000 x g. The enriched cytosolic fraction was finally recovered by centrifuging at 200000 x g and by transferring the supernatant to a clean tube. The final protein concentration in the preparation was 2.5 - 5.0 mg/ml.

*Chromatographic Fractionation*

All fractionation steps were carried out using an ÄKTAexplorer 10 chromatography system (Amersham) at room temperature. The cytosol preparations (10 mg of total protein) were passed through a 0.45 $\mu$m Milex-HV syringe-driven filter unit and the loaded onto desalting columns (three 5 ml HiTrap desalting columns connected in series, Amersham). The proteins were eluted using Buffer A (25 mM NaHPO$_4^-$ pH 7.5; 1 mM EDTA; 0.5 mM dithioerythritol; 1x Complete EDTA-free (Protease inhibitor cocktail tablets from Roche Diagnostics; pH adjusted to 7.5) using a flow rate of 1.5 ml/min. Proteins were recovered in a 20 ml injectionloop using the increase in UV absorption (280 nm) and the minimum in conductivity as boundaries for the protein fraction. The proteins were then separated by anion exchange chromatography using a TSK DEAE-5PW 7.5cmx7.5mm column (TOSOH BIOSEP) at a flow rate of 1 ml/min. Buffer A was used as the binding buffer, buffer B (25 mM NaHPO$_4^-$ pH 7.5; 1 mM EDTA; 0.5 mM dithioerythritol; 1x Complete EDTA-free (Protease inhibitor cocktail tablets from Roche Diagnostics; 1 M NaCl, pH adjusted to 7.5) as the elution buffer. The sample was loaded onto the column and unbound material was washed off with 7 column volumes (CV) of Buffer A. The bound proteins were then eluted by three-segment gradient (1st segment: 0 - 11% Buffer B in 3 CV; 2nd segment: 11 - 30% Buffer B in 10 CV; 30 - 50% Buffer B in 1.5 CV). Finally, the column was washed with 5 CV of 50% Buffer B. Fractions of 1 ml were collected and combined to form eight pools plus the flow-through. The conductivity boundaries were: FT: UV280 increase to increase in conductivity; 1 (start of conductivity-increase to 12 mS); 2 (12 to 15 mS); 3 (15 to 18 mS); 4 (18 to 21 mS); 5 (21 to 24 mS); 6 (24 to 27 mS); 7 (27 to 30 mS); 8 (30 to 40 mS).

*Two-Dimensional Electrophoresis*

The fractions were concentrated and desalted by reversed phase chromatography using self-packed syringe-driven minicolumns (MoBiTec M1002) filled with 100 mg of POROS 20 R1 material (PerSeptive Biosystems). The columns were washed with 10 ml of 0.1% Trifluoroacetic Acid (TFA) and with 70% Acetonitrile/ 0.1% TFA. After loading the sample, the columns were washed with 10 ml of 0.1% TFA and eluted with 2 ml of 70% Acetonitrile/ 0.1% TFA. The eluate was then dried in a SpeedVac evaporator and taken up in IEF Sample Buffer (7 M Urea, 2 M Thiourea, 50 mM Tris pH 7.5, 2 % (w/v) CHAPS, 0.4 % (w/v) Dithioerythritol, 0.5% (w/v) ampholytes). Aliquots containing 0.5 mg of protein were set aside from each fraction and labeled as Sample 1 to 8. The following samples were prepared from the remainder of the fractions: Sample 9: 0.25 mg fraction 1 + 0.25 mg fraction 2; Sample 10: 0.25 mg fraction 3 + 0.25 mg fraction 4; Sample 11: 0.25 mg fraction 5 + 0.25 mg fraction 6; Sample 12: 0.25 mg fraction 7 + 0.25 mg fraction 8; Sample 13: 0.125 mg fraction 1 + 0.125 mg fraction 2+ 0.125 mg fraction 3 + 0.125 mg fraction 4; Sample 14: 0.125 mg fraction 5 + 0.125 mg fraction 6+ 0.125 mg fraction 7 + 0.125 mg fraction 8; Sample 15: 0.0625 mg fraction 1 + 0.0625 mg fraction 2 + 0.0625 mg fraction 3+ 0.0625 mg fraction 4+ 0.0625 mg fraction 5+ 0.0625 mg fraction 6+ 0.0625 mg fraction 7+ 0.0625 mg fraction 8. Thus, samples 1 - 8 contain 0,5 mg of underline protein fractions, samples 9 - 12 each correspond to a two-fold dilution of these samples, samples 13 and 14 to a four-fold, and sample 15 to an eight-fold dilution of these original fractions. Isoelectric Focusing was performed using immobilized pH gradient (IPG) strips with a pH range from 3 to 10 (IPG 3-10L; Amersham) in a Protean IEF Cell (BioRad) at 20°C. The dried strips were re-hydrated in a solution containing 7 M Urea, 2M Thiourea, 2 % (w/v) CHAPS, 0.4 % (w/w) Dithioerythritol, and 0.5 % (w/v) ampholytes. The protein fractions were cup-loaded at the cathodic end of the strip. The voltage was linearly increased to 5000V over 8h, followed by a 5000 V plateau for 10 h. The strips were equilibrated and alkylated by successive washes in Equilibration Solution I (6 M Urea, 50 mM Tris pH 7.5, 30 % Glycerol, 2.0 % SDS, 30 mM Dithioerythritol) and Equilibration Solution II (6 M Urea, 50 mM Tris pH 8.8, 30 % Glycerol, 2.0 % SDS, 0.23 M Iodoacetamide) for 10 min each. The strips were loaded onto 11% Acrylamide/PDA (37:1) gradient gels (240 mm x 200 mm x 1.5 mm). The proteins were resolved by electrophoresis at 80V O/N in an ETTAN Dalt Electrophoresis apparatus (Amersham) with constant cooling (20°C).

*Gel Staining and Processing*

The gels were fixed in 50% methanol/ 10% acetic acid and stained with Coomassie Blue (Colloidal Blue, Invitrogen, Carlsbad, CA) overnight followed by multiple washes in ultra-pure water for 7h total. The gels were scanned and spots with a diameter of 1.2 mm were excised using an automatic spot picking device. The spots were de-stained in a solution containing 100 mM Ammonium hydrogen carbonate and 30 % Acetonitrile. The dried de-stained gel pieces were digested in 5 $\mu$l of a 10 $\mu$g/ml Trypsin solution (Roche Diagnostics) overnight at room temperature. After addition of 10 $\mu$l of ultra-pure water, proteins were extracted with 5 $\mu$l of solution containing 75% Acetonitrile and 0.3% (v/v) TFA. The peptide solution was spotted onto a MALDI target together with $\alpha$-Cyano-4-hydroxycinnamic acid as matrix.

*Mass Spectrometry and Protein Identification*

Peptide masses were measured on a Bruker Ultraflex Instrument (Bruker, Bremen, Germany), using ACTH and Bradykinin as internal mass standards. As explained below, monoisotopic peptide masses were automatically detected from the mass spectra and compared to theoretical masses of peptides derived from an in-silico tryptic digest of all proteins from a database of protein sequences (e.g. SwissProt, or NCBI rat genome draft).

*Peak annotation for MALDI mass spectra*

The mass spectrometric data is two times filtered with a low-pass median parametric spline filter in order to determine the instrument baseline. The smoothed residual mean standard deviation from the baseline is used as an estimate of the instrument noise level in the data.
After baseline correction and rescaling of the data in level-over-noise coordinates, the data point with the largest deviation from the baseline is used to seed a non-linear (Levenberg-Marquardt) data fitting procedure to detect possible peptide peaks. Specifically, the fit procedure attempts to produce the best fitting average theoretical peptide isotope distribution parameterized by peak height, resolution, and monoisotopic mass. The convergence to a significant fit is determined in the usual way by tracking sigma values.
After a successful convergence, an estimate for the errors of the determined parameters is produced using a bootstrap procedure using sixteen repeats with a random exchange of 1/3 of the data points.
The resulting fit is subtracted from the data, the noise level in the vicinity of the fit is adjusted to the sum of the extrapolated noise level and the deviation from the peak fit, and the process is iterated to find the next peak as long as a candidate peak more than five times over level of noise can be found. The process is stopped when more than 50 data peaks have been found.
The zero and first order of the time-of flight to mass conversion are corrected using linear extrapolation from detected internal standard peaks, and confidence intervals for the monoisotopic mass values are estimated form the mass accuracies of the peaks and standards.

*Probabilistic matching of spectra peaks to in-silico protein digests*

Peak mass lists for mass spectra are directly compared to theoretical digests for whole protein sequence databases. For each theoretical digest, $[1-\Pi(1- N\ p(pi))]^{cMatches}$ is calculated, where N is the number of peptides in the digest, P(pi) is the number of peptides that match the confidence interval for the monoisotopic mass of the peak divided by the count of all peptides in the sequence database, and cMatches is the number of matches between digest and mass spectrum. It can be shown that this value is proportional to the probability of obtaining a false positive match between digest and spectrum. Probability values are further filtered for high significance of the spectra peaks that produce the matches. After a first round of identifications, deviations of the identifications for mass spectra acquired under identical conditions are used to correct the second and third order terms of the time-of-flight to mass conversion. The resulting mass values have mostly absolute deviations less than 10ppm. These mass values are then used for a final round of matching, where all matches having a Pmism less than 0.01/NProteins (1% significance level with Bonferoni correction) are accepted.

*Database analysis*

For each protein in the database, the number of identifications per 2D-PAGE gel analyzed in this study was counted. In this example the dilution level 1 was set as reference. Then, the following values were derived:

- Number of identifications in dilution level 1 (undiluted samples, samples 1- 8) = $N_1$

- Number of identifications in dilution level 2 (2-fold dilution, samples 9 - 12) = $N_2$

- Number of identifications in dilution level 3 (4-fold dilution, samples 13,14) = $N_3$

- Number of identifications in dilution level 4 (8-fold dilution, sample 15) = $N_4$

As expected, for most proteins the N values decreased roughly two-fold from layer to layer. To account for the dilution factors and to derive a rough absolute quantity for each protein, a quantity value q was calculated as follows:

$$q = (N_1 + 2 \times N_2 + 4 \times N_3 + 8 \times N_4)\ /$$
$$\text{total number of identified protein spots for all samples of the same source}$$
$$\text{on all dilution levels}$$

The division by the total number of identification for all samples of the same source was introduced to account for inter-sample variations in protein concentration.
For each protein, the q values for both mixture samples (high and low glucose) were calculated and compared.
The following three proteins were chosen as examples for the illustration of the feasibility of this quantification method: Glycogen Phosphorylase (liver form); Vimentin, and Heat shock protein 105 (Table 1, Fig. 3).

Table 1: relative Quantity (q Values) of the proteins present in the cytosol obtained for the three experiments for three example proteins

| | q (5mM Glucose) x $10^{-5}$ | q (10 mM Glucose) x $10^{-5}$ |
|---|---|---|
| Glycogen phosphorylase | | |
| Experiment 1 | 112 | 0 |
| Experiment 2 | 8 | 0 |
| Experiment 3 | 124 | 44 |

(continued)

| Vimentin | | |
|---|---|---|
| Experiment 1 | 0 | 2130 |
| Experiment 2 | 80 | 305 |
| Experiment 3 | 0 | 1758 |
| Heat shock protein 105 | | |
| Experiment 1 | 17 | 13 |
| Experiment 2 | 121 | 39 |
| Experiment 3 | 200 | 89 |

Example 2: Collagen alpha I (IV)

Serum samples from three insulin-resistant and three insulin sensitive patients (Caucasian, female) were fractionated as described below. The Body Mass Index (BMI) and the Glucose Disposal Rate (GDR) as determined by the Euglycemic-Hyperinsulinemic Clamp method (Garvey et al. Diabetes 34 (1985) 222-234) are indicated in Table 2. Combinatorial serial dilution was performed as described in the patent application and the resulting samples were subjected to Two-Dimensional-SDS-Polyacrylamide Gel Electrophoresis (2D-PAGE) as described below. All detectable protein spots were excised from each gel. The proteins were digested with trypsin and the resulting peptides subjected to Matrix-Assisted Laser Desorption Ionization Time-of-Flight Mass Spectrometry (MALDI-MS). Protein identification was achieved by peptide mass fingerprint analysis as described below and protein lists were compared as described in Example 1.

Table 2: Body Mass Index (BMI) and Glucose Disposal Rates (GDR) determined by the euglycemic hyperinsulinemic clamp method of six subjects. As GDRs above 15 are considered as the breakpoint for the determination of insulin resistance, the patients on the left side of the panel are classified as insulin sensitive (IS) and those on the right side as insulin resistant (IR). Plasma from these individuals was analyzed by serial combinatorial dilution followed by 2D-PAGE, spot excision, tryptic digest, MALDI-MS and finally protein identification by peptide mass fingerprint comparison.

| Patient | Insulin-Sensitive (IS) | | Patient | Insulin-Resistant (IR) | |
|---|---|---|---|---|---|
| | BMI | GDR | | BMI | GDR |
| IS1 | 22.4 | 21.9 | IR1 | 31.3 | 10.2 |
| IS2 | 22.4 | 19.7 | IR2 | 33 | 11.65 |
| IS3 | 29.5 | 20.4 | IR3 | 33.1 | 8.0 |

*Sample Preparation*

A method was established to search for Insulin Resistance markers in human plasma by applying proteomics technologies. Plasma is a difficult to analyze by Proteomics techniques because it includes ca. ten high-abundance proteins, which represent approximately 98% of the total protein mass. The high-abundance proteins, albumin and antibody chains were removed, by applying chromatographic techniques and fractionated the flow through fraction over an ion exchanger. The scheme described comprises three chromatography steps, matrix blue, protein G and ion exchange, and is highly reproducible. All chromatographic steps were performed on an FPLC System (Pharmacia).

*Removal of albumin by affinity chromatography on Mimetic blue and removal of immunoglobulins by affinity chromatography on Protein G*

Human plasma was received from three control individuals and three patients with diabetes type II. Protease inhibitors cocktail (Roche Diagnostics, Mannheim, Germany) was added to the plasma (one tablet to 50 ml). Plasma was diluted three-fold with 25 mM MES, pH 6.0, to reduce the salt concentration and adjust the pH to about 6.0. The two columns, Mimetic blue SA P6XL (50 ml, ProMetic BioSciences Ltd.) and HiTrap Protein G HP (5 ml, Amersham Biosciences) were connected in series and equilibrated with 25 mM MES, pH 6.0. The volume corresponding to approximately one g of plasma protein(15 ml, 66 mg/ml) was filtered through a 0.22 μm filter and applied onto the Mimetic blue column at 5 ml/min. The flow through of this column was directly loaded onto the Protein G column and the flow-through fraction from the latter column was collected (about 120 mg). The two columns were washed with 100 ml of 25 mM MES, pH 6.0 and then they were separated. The Mimetic blue column was eluted with a step gradient of 2 M NaCl in 50 mM Tris-HCl, pH 7.5 and the Protein G was eluted with 100 mM glycine-HCl, pH 2.8 and the eluate was neutralized with 1 M Tris base. The flow through fraction and the two eluates were analyzed by two-dimensional gels and the proteins were identified by MALDI-MS. In the eluate from Mimetic blue, mainly full-length and fragmented albumin were detected. In the eluate from the Protein G column, mainly heavy and light Ig chains were detected. Most of the other plasma proteins were recovered in the flow through fraction.

*Protein fractionation by ion exchange chromatography*

The flow through and the wash fractions from the Mimetic blue and Protein G columns were combined, adjusted to pH 8.0 with 2 M Tris base and were applied onto a HiTrap Q HP column (5 ml, Amersham Biosciences), equilibrated with 50 mM Tris-HCl, pH 8.0 at 5 ml/min. The column was eluted with a liner gradient of increasing salt concentration from 0 to 1 M NaCl in 50 mM Tris-HCl, pH 7.5. Five-ml fractions were collected and analyzed by 1-D gels. Approximately 50 mg of total protein were recovered from this column. On the basis of the gel analysis, the fractions were pooled to form eight pools, so that each pool included about 5 mg of total protein. The pools were concentrated with Ultrafree-15 Centrifugal Filter (5k MWCO, Millipore) and each of the eight pools was analyzed by 2-D gels. About 400 spots from each gel were excised and analyzed by MALDI-MS.

*2D-PAGE*

Immobilized pH gradient (IPG) strips were purchased from Amersham Biosciences (Uppsala, Sweden). Acrylamide was obtained from Biosolve (Valkenswaard, The Netherlands) and the other reagents for the polyacrylamide gel preparation were from Bio-Rad Laboratories (Hercules, CA, USA). CHAPS was from Roche Diagnostics (Mannheim, Germany), urea from Applichem (Darmstadt, Germany), thiourea from Fluka (Buchs, Switzerland) and dithioerythritol from Merck (Darmstadt).

Samples of 0.5 mg total protein were applied on 3-10 NL IPG strips, in sample cups at their basic and acidic ends. Focusing started at 200 V, and the voltage was gradually increased to 5000 V at 3 V/min, using a computer-controlled power supply and was kept constant for a further 6 h. The second-dimensional separation was performed either on 12% constant SDS polyacrylamide gels (180x200x1.5 mm) at 40 mA per gel. After protein fixation for 12 h in 40% methanol that contained 5% phosphoric acid, the gels were stained with colloidal Coomassie blue (Novex, San Diego, CA, USA) for 24 h. Excess dye was washed from the gels with $H_2O$, and the gels were scanned in an Agfa DUOSCAN densitometer (resolution 400). Electronic images of the gels were recorded with Photoshop (Adobe) software. The images were stored in tiff (about 5 Mbytes/file) and jpeg (about 50 Kbytes/file) formats. The gels were kept at 4°C until used for MS analysis.

*MALDI-MS*

Selected spots of 1.2 mm diameter were excised with a homemade spot picker (described in European Application EP 1 384 994), placed into 96-well microtiter plates and each gel piece was destained with 100 μl of 30% acetonitrile in 50 mM ammonium bicarbonate in a CyBi™-Well apparatus (Cybio AG, Jena, Germany). After destaining, the gel pieces were washed with 100 μl of $H_2O$ for 5 min, and dried in a speedvac evaporator without heating for 45 min. Each dried gel piece was rehydrated with 5 μl of 1 mM ammonium bicarbonate, that contained 50 ng trypsin (Roche Diagnostics, Mannheim, Germany). After 16 h at room temperature, 20 μl of 50% acetonitrile, that contained 0.3% trifluoroacetic acid was added to each gel piece. The gel pieces were incubated for 15 min with constant shaking. A peptide mixture (1.5 μl) was simultaneously applied with 1 μl of matrix solution, that consisted of 0.025% α-cyano-4-hydroxycinnamic acid (Sigma), and that contained the standard peptides des-Arg-bradykinin (Sigma, 20 nM, 904.4681 Da) and adrenocorticotropic hormone fragment 18-39 (Sigma, 20 nM, 2465.1989 Da) in 65% ethanol, 32% acetonitrile, and 0.03% trifluoroacetic acid, to the AnchorChip™. The sample application was performed with a CyBi-Well apparatus. Samples were analyzed in a time-of-flight mass spectrometer (Ultraflex TOF-TOF, Bruker Daltonics) in the reflectron mode. An accelerating voltage of 20 kV was used. Proteins were identified on the basis of peptide-mass matching.

*Peak annotation for MALDI mass spectra*

Mass spectrometric data is two times filtered using a low-pass median parametric spline filter in order to determine the instrument baseline. The smoothed residual mean standard deviation from the baseline is used as an estimate of the instrument noise level in the data. After baseline correction and rescaling of the data in level-over-noise coordinates, the data point with the largest deviation from the baseline is used to seed a non-linear (Levenberg-Marquardt) data fitting procedure to detect possible peptide peaks. Specifically, the fit procedure attempts to produce the best fitting average theoretical peptide isotope distribution parameterized by peak height, resolution, and monoisotopic mass. The convergence to a significant fit is determined in the usual way by tracking sigma values. After a successful convergence, an estimate for the errors of the determined parameters is produced using a bootstrap procedure using sixteen repeats with a random exchange of 1/3 of the data points. The resulting fit is subtracted from the data, the noise level in the vicinity of the fit is adjusted to the sum of the extrapolated noise level and the deviation from the peak fit, and the process is iterated to find the next peak as long as a candidate peak more than five times over level of noise can be found. The process is stopped when more than 50 data peaks have been found. The zero and first order of the time-of flight to mass conversion are corrected using linear extrapolation from detected internal standard peaks, and confidence intervals for the monoisotopic mass values are estimated form the mass accuracies of the peaks and standards.

*Probabilistic matching of spectra peaks to in-silico protein digests*

Peak mass lists for mass spectra are directly compared to theoretical digests for whole protein sequence databases. For each theoretical digest, $[1-\Pi(1- N P(pi))]^{cMatches}$ is calculated, where *N* is the number of peptides in the theoretical digest, *P(pi)* is the number of peptides that match the confidence interval for the monoisotopic mass of the peak divided by the count of all peptides in the sequence database, and *cMatches* is the number of matches between digest and mass spectrum. It can be shown that this value is proportional to the probability of obtaining a false positive match between digest and spectrum. Probability values are further filtered for high significance of the spectra peaks that produce the matches. After a first round of identifications, deviations of the identifications for mass spectra acquired under identical conditions are used to correct the second and third order terms of the time-of-flight to mass conversion. The resulting mass values have mostly absolute deviations less than 10ppm. These mass values are then used for a final round of matching, where all matches having a $P_{mism}$ less than 0.01/NProteins (1% significance level with Bonferoni correction) are accepted.

*Results*

Collagen alpha I (IV) (Collagen IV; Swissprot accession numbers P12109; 000117; 000118; Q14040; Q14041; Q16258) was exclusively detected in two insulin resistant patients (IR2 and IR3, see Table 3). In one patient (IR2), the spots were detected at the second level (two-fold diluted sample), whereas in the second patient (IR3), the protein was detected twice at the forth level (eightfold combinatorial dilution). The number of identifications was multiplied with the dilution factor (in this case, one and four, respectively) and corrected for the total number of protein spots identified for the respective sample.

Collagen IV levels were also measured using an immunoassay (Biotrin Collagen IV EIA; Catalogue Number NoBIO82; Biotrin, Dublin, Ireland) following the supplier's protocol. The results from the two assays are compared in Table 3.

Table 3: Comparison of the results from the serial combinatorial dilution with the results from the immunoassay.

| Patient | IS1 | IS2 | IS3 | IR1 | IR2 | IR3 |
|---|---|---|---|---|---|---|
| Serial combinatorial dilution | 0 | 0 | 0 | 0 | 10 | 1 |
| Collagen IV EIA (ng/ml) | 108 | 111 | 139 | 86 | 208 | 158 |

IS = Insulin-sensitive patient, IR= Insulin-resistant patient.
Serial combinatorial dilution: The number of identifications were adjusted for dilution factor and total spot count.
Immunoassay (Collagen IV EIA): The Collagen IV levels were determined by the Biotrin Collagen IV EIA was used.
The presented results are the mean of duplicate measurements.

The limit of detection of the described proteomic methodology lies above that for the immunoassay at approx. 150 ng/ml. Above that level, proteins can be detected and coarsely quantified by serial combinatorial dilution coupled to the described identification method. Although no absolute quantification is observed, there is some rank correlation, i.e. the samples with the highest and second highest levels were correctly identified.

Serial combinatorial dilution combined with Proteomics type large scale protein identification is an efficient tool to quantify hundreds of proteins in parallel and to identify proteins with marked differences in concentration which can be used in differential protein expression analysis, e.g. for biomarker identification studies.

**Claims**

1. A method for the quantification of a biomolecule in a complex mixture of biomolecules comprising

   a providing at least two fractions of a fractionation of a mixture of biomolecules comprising each at least one distinct component,
   b subjecting the fractions to a serial combinatorial dilution step, until the concentrations of the biomolecule fall below the sensitivity threshold of the subsequent detection and identification method
   c detecting and identifying the biomolecule in each original fraction and each diluted fraction by a method providing a sensitivity threshold and identity information to determine the presence or absence of said biomolecule in each of the original and diluted fractions, and
   d quantifying the biomolecule in the complex mixture of biomolecules by summarizing of the number of identifications of the biomolecule in each fraction on each dilution level in consideration of the respective dilution factor.

2. A method according to claim 1, wherein the sum of d) is divided by the total number of identifications of all biomolecules in all fractions on all dilution levels.

3. A method according to claim 1 or 2 wherein the number of fractions of a) depends on the complexity of the mixture of biomolecules.

4. A method according to any one of the claims 1 to 2 wherein the number of fractions of a) depends on the concentration of the different biomolecules in the complex mixture of biomolecules.

5. A method according to any one of the claims 1 to 2 wherein the number of fractions of a) depends on the detection and identification method of c)

6. A method according to any one of the claims 1 to 5 wherein the number of dilution levels of b) depends on the starting concentration of the biomolecules in the fractions.

7. A method according to any one of the claims 1 to 5 wherein the number of dilution levels of b) depends on the number of fractions after fractionation.

8. A method according to any one of the claims 1 to 5 wherein the number of dilution levels of b) depends on the detection limit of the detection and identification method.

9. A method according to any one of the claims 1 to 8 wherein the biomolecule is present in not more than n minus one fractions wherein n is the total number of fractions and wherein n is equal or higher than two.

10. A method according to any one of the claims 1 to 8 wherein the biomolecule is present in two fractions.

11. A method according to any one of the claims 1 to 8 wherein the biomolecule is present in one fraction.

12. A method according to any one of the claims 1 to 11 wherein the detecting and identifying method of c) comprises a two dimensional gel electrophoresis.

13. A method according to claim 12 wherein the detecting and identifying method of c) comprises additionally mass spectrometry.

14. A method according to any one of the claims 1 to 11 wherein the detecting and identifying method of c) comprises immunoassays.

15. A method according to any one of the claims 1 to 11 wherein the detecting and identifying method of c) comprises gas chromatography combined with mess spectrometry.

16. A method according to any one of the claims 1 to 11 wherein the detecting and identifying method of c) comprises electrophoresis with specifically labeled molecular entities.

17. A method according to any one of the claims 1 to 16 wherein the fractions of a) are separated by chromatographic fractionation, ultracentrifugation, protein precipitation, or immunoprecipitation.

18. A method according to any one of the claims 1 to 17 wherein the biomolecule is a polypeptide.

19. A method according to any one of the claims 1 to 17 wherein the biomolecule is polynucleotide.

20. A method according to any one of the claims 1 to 17 wherein the biomolecule is a carbohydrate.

21. A method according to any one of the claims 1 to 17 wherein the biomolecule is a lipid.

22. A method according to any one of the claims 1 to 17 wherein the biomolecule is a glycoprotein.

23. A method according to any one of the claims 1 to 17 wherein the biomolecule is a lipoprotein.

**Patentansprüche**

1. Verfahren zur quantitativen Bestimmung eines Biomoleküls in einem komplexen Gemisch von Biomolekülen, umfassend

   a. das Bereitstellen von zumindest zwei Fraktionen einer Fraktionierung eines Gemisches von Biomolekülen, die jeweils mindestens eine unterscheidbare Komponente umfassen,
   b. das Unterziehen der Fraktionen einem kombinatorischen Reihenverdünnungsschritt bis die Konzentrationen des Biomoleküls unter die Empfindlichkeitsschwelle des anschließenden Detektions- und Identifikationsverfahrens fallen,
   c. das Detektieren und Identifizieren des Biomoleküls in jeder Ausgangsfraktion und jeder verdünnten Fraktion durch ein Verfahren, das eine Empfindlichkeitsschwelle und Identitätsinformationen zur Bestimmung der Gegenwart oder Abwesenheit des Biomoleküls in jeder der Ausgangs- und verdünnten Fraktionen liefert, und
   d. das Quantifizieren des Biomoleküls in dem komplexen Gemisch von Biomolekülen durch Zusammenfassen der Anzahl an Identifikationen des Biomoleküls in jeder Fraktion bei jedem Verdünnungsgrad unter Berücksichtigung des jeweiligen Verdünnungsfaktors.

2. Verfahren nach Anspruch 1, wobei die Summe von d) durch die Gesamtanzahl von Identifikationen aller Biomoleküle in allen Fraktionen bei allen Verdünnungsgraden geteilt wird.

3. Verfahren nach Anspruch 1 oder 2, wobei die Anzahl der Fraktionen von a) von der Komplexität des Gemisches von Biomolekülen abhängt.

4. Verfahren nach einem der Ansprüche 1 bis 2, wobei die Anzahl der Fraktionen von a) von der Konzentration der unterschiedlichen Biomoleküle in dem komplexen Gemisch von Biomolekülen abhängt.

5. Verfahren nach einem der Ansprüche 1 bis 2, wobei die Anzahl der Fraktionen von a) von dem Detektions- und Identifikationsverfahren von c) abhängt.

6. Verfahren nach einem der Ansprüche 1 bis 5, wobei die Anzahl der Verdünnungsgrade von b) von der Ausgangskonzentration der Biomoleküle in den Fraktionen abhängt.

7. Verfahren nach einem der Ansprüche 1 bis 5, wobei die Anzahl der Verdünnungsgrade von b) von der Anzahl der Fraktionen nach der Fraktionierung abhängt.

8. Verfahren nach einem der Ansprüche 1 bis 5, wobei die Anzahl der Verdünnungsgrade von b) von der Erfassungsgrenze des Detektions- und Identifikationsverfahrens abhängt.

9. Verfahren nach einem der Ansprüche 1 bis 8, wobei das Biomolekül bei nicht mehr als n minus eine Fraktion vorliegt, wobei n die Gesamtanzahl an Fraktionen ist und wobei n gleich oder höher als zwei ist.

10. Verfahren nach einem der Ansprüche 1 bis 8, wobei das Biomolekül in zwei Fraktionen vorliegt.

**11.** Verfahren nach einem der Ansprüche 1 bis 8, wobei das Biomolekül in einer Fraktion vorliegt.

**12.** Verfahren nach einem der Ansprüche 1 bis 11, wobei das Detektions- und Identifikationsverfahren von c) eine zweidimensionale Gelelektrophorese umfasst.

**13.** Verfahren nach Anspruch 12, wobei das Detektions- und Identifikationsverfahren von c) außerdem Massenspektrometrie umfasst.

**14.** Verfahren nach einem der Ansprüche 1 bis 11, wobei das Detektions- und Identifikationsverfahren von c) Immunoassays umfasst.

**15.** Verfahren nach einem der Ansprüche 1 bis 11, wobei das Detektions- und Identifikationsverfahren von c) Gaschromatographie in Kombination mit Massenspektrometrie umfasst.

**16.** Verfahren nach einem der Ansprüche 1 bis 11, wobei das Detektions- und Identifikationsverfahren von c) Elektrophorese mit speziell markierten molekularen Einheiten umfasst.

**17.** Verfahren nach einem der Ansprüche 1 bis 16, wobei die Fraktionen von a) durch chromatographische Fraktionierung, Ultrazentrifugation, Proteinfällung oder Immunfällung getrennt werden.

**18.** Verfahren nach einem der Ansprüche 1 bis 17, wobei das Biomolekül ein Polypeptid ist.

**19.** Verfahren nach einem der Ansprüche 1 bis 17, wobei das Biomolekül ein Polynucleotid ist

**20.** Verfahren nach einem der Ansprüche 1 bis 17, wobei das Biomolekül ein Kohlenhydrat ist.

**21.** Verfahren nach einem der Ansprüche 1 bis 17, wobei das Biomolekül ein Lipid ist.

**22.** Verfahren nach einem der Ansprüche 1 bis 17, wobei das Biomolekül ein Glycoprotein ist.

**23.** Verfahren nach einem der Ansprüche 1 bis 17, wobei das Biomolekül ein Lipoprotein ist.


**Revendications**

**1.** Méthode pour la quantification d'une biomolécule dans un mélange complexe de biomolécules, comprenant les étapes consistant à

   a. fournir au moins deux fractions d'un fractionnement d'un mélange de biomolécules comprenant chacune au moins un constituant distinct,
   b. soumettre les fractions à une étape de dilution combinatoire en série, jusqu'à ce que les concentrations de la biomolécule tombent au-dessous du seuil de sensibilité de la méthode de détection et d'identification ultérieure,
   c. détecter et identifier la biomolécule dans chaque fraction initiale et chaque fraction diluée par une méthode fournissant un seuil de sensibilité et une information d'identité pour déterminer la présence ou l'absence de ladite biomolécule dans chacune des fractions initiale et diluée, et
   d. quantifier la biomolécule dans le mélange complexe de biomolécules par récapitulation du nombre d'identifications de la biomolécule dans chaque fraction à chaque niveau de dilution en prenant en considération le facteur de dilution respectif.

**2.** Méthode suivant la revendication 1, dans laquelle la somme de d) est divisée par le nombre total d'identifications de toutes les biomolécules dans toutes les fractions à tous les niveaux de dilution.

**3.** Méthode suivant la revendication 1 ou 2, dans laquelle le nombre de fractions de a) dépend de la complexité du mélange de biomolécules.

**4.** Méthode suivant l'une quelconque des revendications 1 et 2, dans laquelle le nombre de fractions de a) dépend de la concentration des différentes biomolécules dans le mélange complexe de biomolécules.

**5.** Méthode suivant l'une quelconque des revendications 1 et 2, dans laquelle le nombre de fractions de a) dépend de la méthode de détection et d'identification de c).

**6.** Méthode suivant l'une quelconque des revendications 1 à 5, dans laquelle le nombre de niveaux de dilution de b) dépend de la concentration de départ des biomolécules dans les fractions.

**7.** Méthode suivant l'une quelconque des revendications 1 à 5, dans laquelle le nombre de niveaux de dilution de b) dépend du nombre de fractions après fractionnement.

**8.** Méthode suivant l'une quelconque des revendications 1 à 5, dans laquelle le nombre de niveaux de dilution de b) dépend de la limite de détection de la méthode de détection et d'identification.

**9.** Méthode suivant l'une quelconque des revendications 1 à 8, dans laquelle la biomolécule est présente dans pas plus de n moins un fractions, n étant le nombre total de fractions et n étant égal ou supérieur à 2.

**10.** Méthode suivant l'une quelconque des revendications 1 à 8, dans laquelle la biomolécule est présente dans deux fractions.

**11.** Méthode suivant l'une quelconque des revendications 1 à 8, dans laquelle la biomolécule est présente dans une fraction.

**12.** Méthode suivant l'une quelconque des revendications 1 à 11, dans laquelle la méthode de détection et d'identification de c) comprend une électrophorèse sur gel bidimensionnelle.

**13.** Méthode suivant la revendication 12, dans laquelle la méthode de détection et d'identification de c) comprend en outre une spectrométrie de masse.

**14.** Méthode suivant l'une quelconque des revendications 1 à 11, dans laquelle la méthode de détection et d'identification de c) comprend des analyses immunologiques.

**15.** Méthode suivant l'une quelconque des revendications 1 à 11, dans laquelle la méthode de détection et d'identification de c) comprend la chromatographie en phase gazeuse combinée avec la spectrométrie de masse.

**16.** Méthode suivant l'une quelconque des revendications 1 à 11, dans laquelle la méthode de détection et d'identification de c) comprend une électrophorèse avec des entités moléculaires marquées spécifiquement.

**17.** Méthode suivant l'une quelconque des revendications 1 à 16, dans laquelle les fractions de a) sont séparées par fractionnement chromatographique, ultracentrifugation, précipitation des protéines ou immunoprécipitation.

**18.** Méthode suivant l'une quelconque des revendications 1 à 17, dans laquelle la biomolécule est un polypeptide.

**19.** Méthode suivant l'une quelconque des revendications 1 à 17, dans laquelle la biomolécule est un polynucléotide

**20.** Méthode suivant l'une quelconque des revendications 1 à 17, dans laquelle la biomolécule est un glucide.

**21.** Méthode suivant l'une quelconque des revendications 1 à 17, dans laquelle la biomolécule est un lipide.

**22.** Méthode suivant l'une quelconque des revendications 1 à 17, dans laquelle la biomolécule est une glycoprotéine.

**23.** Méthode suivant l'une quelconque des revendications 1 à 17, dans laquelle la biomolécule est une lipoprotéine.

Fig. 1

Fig. 2

$$q = \frac{\sum (d_i \times N_i)}{N_{total}}$$

Fig. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1384994 A

**Non-patent literature cited in the description**

- *European Journal Biochemistry,* 1996, vol. 240, 756-764

- **Garvey et al.** *Diabetes,* 1985, vol. 34, 222-234